# EUROPEAN PATENT APPLICATION

(11) **EP 1 684 072 A1**
(43) Date of publication of application: **26.07.2006**
(21) Application number: 04799737.4
(22) Date of filing: 11.11.2004
(51) Int. Cl.: G01N 33/53, G01N 37/00

(54) **BIOASSAY SUBSTRATE ON WHICH FEEDER WIRING IS LAID**

(30) Priority: 11.11.2003 JP 2003380787
(71) Applicant: SONY CORPORATION, Tokyo 141-0001 (JP)
(72) Inventor: YOSHIO, Akira, (JP)
(74) Representative: Keston, Susan Elizabeth
(86) International application number: PCT/JP2004/017124
(87) International publication number: WO 2005/050205

(57) **Abstract**

A contrivance is applied to the wiring configuration for securely feeding electricity to electrodes provided in a multiplicity of reaction regions arranged on a substrate, irrespectively of the arrangement configuration of the electrodes. A bioassay substrate being a disk-shaped substrate having such a configuration that reaction regions 2 to be fields for interactions between substances are arranged in multiplicity and electrodes E are provided in the reaction regions 2, wherein feeder wirings 401, 402 which are extended from a current passing portion or portions (for example, 36) provided at a central portion of the substrate 1 and which are connected to the electrodes E1, E2 in the reaction regions 2 are extended to entirely cover the whole area of the substrate.

## Description

### Technical Field

The present invention relates to a bioassay substrate for DNA chips or the like which is composed of a disk-shaped substrate.

Specifically, the invention relates to the technology pertaining to the configuration of feeder wirings extended from a current passing portion provided at a predetermined position of a disk-shaped substrate to electrodes in respective reaction regions arranged on the substrate.

### Background Art

A first conventional technology pertaining to the present invention is the technology concerning a bioassay integrated substrate called a DNA chip or DNA microarray (hereinafter generically referred to as "DNA chip") in which predetermined DNAs are microscopically arranged by the microarray technique. The DNA chip technology is configured so that it is possible to comprehensively analyze inter-molecular reactions such as hybridization because a variety of and multiplicity of DNA oligo chains or cDNAs (complementary DNAs) are integrated on a glass substrate or silicon substrate. Therefore, the DNA chips have been utilized for gene mutation analysis, SNPs (Single Nucleotide Polymorphisms) analysis, gene expression frequency analysis, etc., and have begun to be widely used in novel drug development, clinical diagnosis, pharmaceutical genomics, legal medicine and other fields. In addition to the DNA chips, there have also been developed protein chips having proteins fixed on substrates, biosensors for analyzing various inter-substance interactions, and the like.

A second technology pertaining to the present invention is the technology concerning the actions of an electric field on substances present in an electrically charged state in a liquid phase. Specifically, a nucleotide chain (nucleic acid molecule) is known to extend or migrate under the action of an electric field in a liquid phase. The principle of this phenomenon is considered as follows. It is considered that the phosphate ion (negative charge) constituting the skeleton of the nucleotide chain and the hydrogen atom (positive charge) formed through ionization of water in the vicinity of the phosphate ion form an ion clouding, and polarization vectors (dipoles) generated by these negative and positive charges are as a whole set in one direction when a high-frequency high voltage is impressed thereon, resulting in stretching of the nucleotide chain; in addition, where an uneven electric field with electric lines of force concentrated on an area is impressed, the nucleotide chain migrates toward the location where the electric lines of force are concentrated (see Seiichi Suzuki, Takeshi Yamanashi, Shin-ichi Tazawa, Osamu Kurosawa and Masao Washizu: "Quantitative analysis on electrostatic orientation of DNA in stationary AC electric field using fluorescence anisotropy", IEEE Transaction on Industrial Applications, Vol. 34, No. 1, pp.75 to 83(1998)). Besides, when a DNA solution is placed between micro-electrodes having a gap of several tens to several hundreds of micrometers and a high-frequency electric field of about 1 MV/m and 1 MHz is impressed thereon, dielectric polarization occurs in the DNA being present in a random coil form, resulting in that the DNA molecule is stretched rectilinearly in parallel to the electric field. It is known that, under this electrodynamic effect called dielectric migration, the polarized DNA is spontaneously attracted to the electrode end, to be fixed with its one end in contact with the electrode edge (see Masao Washizu, "MINAGARA OKONAU DNA HANDORINGU (DNA Handling under Monitoring)", KASHIKA JOHO, Vol. 20, No. 76 (January 2000)).

The DNA chip technology at present has been spreading as a technology in which a multiplicity of reaction regions for providing the sites of interactions between substances in a liquid phase are preliminarily set on a substrate, and detecting nucleotide chains such as DNA probes are preliminarily fixed in the reaction regions so as to comprehensively analyze the hybridizations which are interactions between the detecting nucleotide chains and complementary target nucleotide chains. In the case of carrying out the DNA chip technology, it is considered that if the detecting nucleotide chains (for example, DNA probes) can be fixed not in the rounded random coil form but in a stretched state in the reaction regions, the bad influences of the so-called steric hindrance and the interferences (for example, adhesion and contact) between the detecting nucleotide chains and the surrounding surfaces, which might arise from the higher-order structures of substances, are excluded and, therefore, the efficiency of the hybridizations is enhanced.

Based on this novel idea, the present inventors have novelly devised a configuration in which an electrode functioning as a detecting surface is preliminarily disposed, and an electric field is impressed on a liquid phase in a reaction region between the electrode and an electrode opposed thereto. Then, the present inventors have successfully established a technology in which by this configuration it is possible to stretch the detecting nucleotide chain present in the random coil form in the liquid phase by the action of the high-frequency electric field, to fix a terminal portion of the detecting nucleotide chain to the electrode edge, and to permit the hybridizations to progress efficiently.

However, in practicing this technology, a means to pass electric currents to the multiplicity of electrodes arranged on the substrate is indispensable. Therefore, in the case of adopting a disk-shaped substrate on which a multiplicity of reaction regions having electrodes can be arranged in various arrangement forms, there arises a problem that a multiplicity of wirings for feeding electricity to the electrodes must be orderly laid on the substrate so that they will not interfere with each other.

In addition, in such a disk-shaped substrate, a special form in which the reaction regions are arranged in the circumferential direction or in a radial pattern is necessarily adopted, so that it is required to devise a wiring configuration more suited to such an arrangement form, specifically, a wiring configuration in which the reaction regions can be arranged over the whole area on the substrate as evenly as possible and in a high density, and to make a contrivance for reducing the wiring resistance.

Besides, at the time of reading the recorded information from the disk-shaped substrate on which the multiplicity of reaction regions are arranged, application of a rotation synchronizing servo or a tracking servo is presumed to be carried out in the same manner as in an operation of reading recorded information from an optical disk such as a CD. Therefore, it is necessary to ensure that exclusive-use signals or marks capable of being utilized for such servos can be read from the substrate, and the configuration on the substrate would thereby be made more complicated. Thus, there is a need to develop a technology by which this problem can be solved.

In view of the foregoing, a primary object of the present invention, for solving the above-mentioned problem, is to provide a disk-shaped bioassay substrate in which a contrivance is applied to the configuration of feeder wirings.

### Disclosure of Invention

According to the present invention, first, there is provided a bioassay substrate being a disk-shaped substrate having such a configuration that reaction regions to be fields for interactions between substances are arranged and electrodes are provided in the reaction regions. In the bioassay substrate, feeder wirings are extended from a current passing portion provided at a central portion of the substrate, and are connected to the electrodes.

In the bioassay substrate having the above configuration, the position of a central portion of the substrate having the disk-shaped form is selected, and the "current passing portion" functioning as a portion where an electric current from an external power supply is supplied. In the present invention, the current passing portion is utilized as a common current passing portion for supplying electric currents to all the feeder wirings extended toward the respective electrodes in the reaction regions arranged in a predetermined pattern over the whole area of the substrate. Incidentally, the current passing portion can be formed, for example, in a circular or ring-like form, and may be in the form of a single current passing region or in the form of being divided into a plurality of independent partial current passing regions.

The feeder wirings are each composed of a first wiring connected to the single current passing region or the partial current passing region of the current passing portion and extended toward the side of the outer circumference (of the substrate), and a second wiring branched and led out from the first wiring. The first wiring can be extended, for example, radially from the current passing portion. In addition, the first wiring may be in the shape of a straight line form wiring or a curved line form wiring or a combination of these shapes, according to the pattern in which the reaction regions are arranged on the substrate, or the like.

The second wirings branched from the first wirings are configured to extend in the circumferential direction, so that the second wirings can be arranged over the whole area of the substrate. The second wirings can be arranged in the form of concentric circles or a spiral line, as viewed from the upper side, by appropriate selection. In addition, there may be freely adopted a wiring configuration in which the second wirings are led out alternately from the adjacent first wirings, a configuration in which the second wirings are connected to only one first wiring, and a configuration in which the second wirings are connected to a plurality of the first wirings.

Here, in the present invention, the second wirings may be used as references for a rotation synchronizing signal or a tracking signal at the time of reading the recorded information on the substrate, whereby the need to provide separate signal references on the substrate is eliminated, thereby enabling simplification of the configuration or structure of the substrate.

The current passing portion provided at a central portion of the substrate may be preliminarily provided in its central portion with a hole having a predetermined aperture diameter, and, further, the hole may be preliminarily provided with a positioning portion for a current passing jig which is to be inserted in the hole so as to supply an electric current to the current passing portion. Furthermore, the hole may be preliminarily provided with a positioning portion for determining the position in the circumferential direction of a chucking jig to be inserted in the hole; these positioning portions may be provided in a combined form.

As the positioning portion, there may be adopted a recess or projection formed in the hole. Besides, the position or positions in the circumferential direction of the current passing jig and/or the chucking jig to be inserted in the hole may be determined by the shape of the hole.

The feeder wirings as above-mentioned may be formed of a plurality of wiring layers. In addition, a configuration may be adopted in which the feeder wirings extended in the plurality of wiring layers are exposed to front on the current passing portion, and the exposed wiring terminal portions are connected to the current passing portion.

In addition, there may also be adopted a configuration in which at least one of the plurality of wiring layers is formed of an electrode layer which is uniform over the whole area, and a configuration in which at least one of the plurality of wiring layers is covered with an insulation layer such as an oxide layer.

Furthermore, there may be adopted a configuration in which the wiring layer located on the side where an excitation beam for reading the interactions in the reaction regions is incident is composed of a conductive film transparent or semi-transparent to rays in the excitation beam wavelength region. The conductive film may be, for example, one selected from the group consisting of an ITO (Indium Tin Oxide) film, a π electron system conductive polymer film, and a metallic thin film having a thickness of not more than 50 *µ*m.

### Brief Description of Drawings

Fig. 1 is an outlook perspective view schematically showing the configuration of a typical embodiment of a bioassay substrate (1) according to the present invention.
Fig. 2 is a plan view schematically showing the basic configuration of wiring in a first embodiment (1a) of a wiring substrate (or wiring layer) used in the present invention.
Fig. 3 is a plan view schematically showing the basic configuration of wiring in a second embodiment (1b) of the wiring substrate (or wiring layer) used in the present invention.
Fig. 4 is a plan view schematically showing the basic configuration of wiring in a third embodiment (1c) of the wiring substrate (or wiring layer) used in the present invention.
Fig. 5 is a plan view schematically showing the basic configuration of wiring in a fourth embodiment (1d) of the wiring substrate (or wiring layer) used in the present invention.
Fig. 6 shows a modified embodiment of the wiring substrate (1d) .
Fig. 7 is a plan view schematically showing the basic configuration of wiring in a fifth embodiment (1e) of the wiring substrate (or wiring layer) used in the present invention.
Fig. 8 is a plan view schematically showing the basic configuration of wiring in a sixth embodiment (1f) of the wiring substrate (or wiring layer) used in the present invention.
Fig. 9 is a plan view schematically showing the basic configuration of wiring in a seventh embodiment (1g) of the wiring substrate (or wiring layer) used in the present invention.
Fig. 10 is a plan view schematically showing the basic configuration of wiring in an eighth embodiment (1h) of the wiring substrate (or wiring layer) used in the present invention.
Fig. 11 is a plan view schematically showing the basic configuration of wiring in a ninth embodiment (1i) of the wiring substrate (or wiring layer) used in the present invention.
Fig. 12 is a plan view schematically showing the basic configuration of wiring in the third embodiment (1j) of the wiring substrate (or wiring layer) used in the present invention.
Fig. 13 is a plan view schematically showing the basic configuration of wiring in the third embodiment (1k) of the wiring substrate (or wiring layer) used in the present invention.
Fig. 14 shows the manner in which an information recording substrate (1) and a wiring substrate (wiring layer), both shown schematically, are laminated integrally to form one bioassay substrate.
Fig. 15 is a plan view, as viewed from the upper side of the substrate (1), of the condition where the substrate (1) and the wiring substrate (1h) are laminated.
Fig. 16 is a vertical sectional view showing an example of connection of feeder wirings to opposed electrodes (E1, E2) arranged in a reaction region (2).
Fig. 17 is a vertical sectional view of a peripheral portion of a reaction region (2) in an embodiment in which a plurality of wiring layers are provided.

### Best Mode for Carrying out the Invention

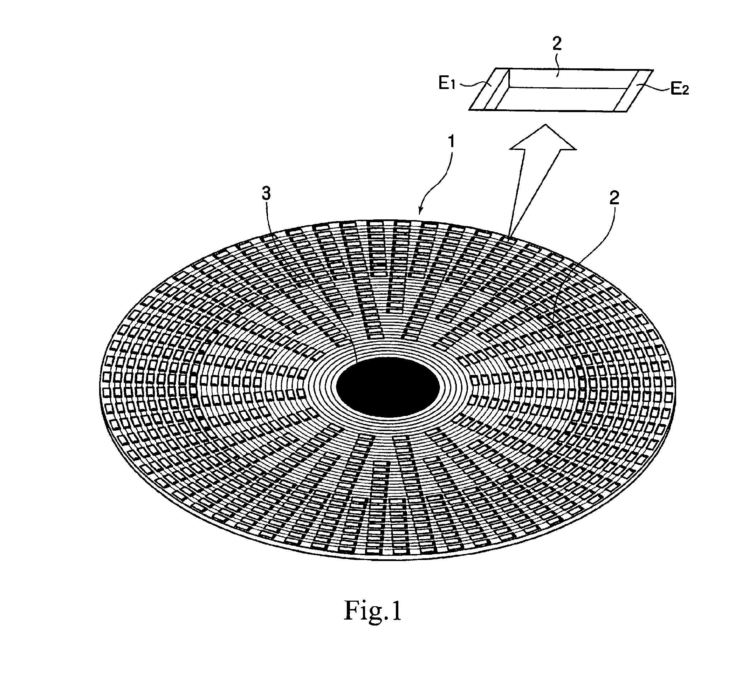

Now, a preferred mode for carrying out the present invention will be described below referring to the accompanying drawings. First, Fig. 1 is an outlook perspective view schematically showing the configuration of a typical embodiment of a bioassay substrate according to the present invention.

In Fig. 1, symbol 1 denotes the typical embodiment of the bioassay substrate (hereinafter referred to simply as "the substrate") according to the present invention. The substrate 1 is formed of an insulation member of a synthetic resin, glass or the like, and is configured to be disk-shaped in form, as viewed from above. On the substrate 1, a multiplicity of reaction regions 2 are arranged radially, in the circumferential direction, or spirally, so that they can be grouped.

The reaction regions 2 are minute regions for providing sites for interactions (for example, hybridization) between substances, and generally have a well shape (recess shape) such that they can reserve or hold a liquid phase, a gel or the like. For deeper understanding, an enlarged view of one of the reaction regions 2 located in the vicinity of the outer circumference of the substrate 1 is added to Fig. 1.

As shown in the view added to Fig. 1, electrodes such as opposed electrodes E1 and E2, for example, are arranged in each of the reaction regions 2 in a required number and at required locations, and a high-frequency AC electric field, a DC electric field or the like is appropriately selectedly impressed on the reaction region 2 through the opposed electrodes E1 and E2, according to the purpose. Incidentally, the arrangement configuration of the electrodes E1 and E2 and the shape of the reaction region 2 are not limited to those shown in Fig. 1.

A configuration may be adopted in which a detecting substance such as a DNA probe can be fixed to a surface portion (for example, an electrode surface) in the reaction region 2. In an exemplary case of using a probe DNA for detection of hybridization, a fixing method may be adopted in which the surface of one electrode (for example, the electrode E1) and a terminal end of the probe DNA are fixed by a reaction such as a coupling reaction.

For example, an electrode surface obtained through a surface treatment with streptoavidin, it is suitable for fixation of a biotinized terminal end of a probe DNA. Or, an electrode surface obtained through a surface treatment with a thiol (SH) group is suitable for fixation of a thiol group-terminated probe DNA through a disulfide linkage (-S-S- linkage).

Next, the substrate 1 is provided at its central portion with a current passing portion 3 having a circular shape or other form. The current passing portion 3 functions as a feeder point or feeder region for contact with a current passing jig (not shown) connected to an external power supply, and plays the role of feeding electricity to the group of the electrodes E1 and E2 in the reaction regions 2 through feeder wirings (not shown in Fig. 1) provided in a wiring configuration which will be described later. Incidentally, the current passing portion 3 is not limited to the circular shape as shown in the figure; for example, the current passing portion 3 may be formed in a ring-like shape or divided shape.

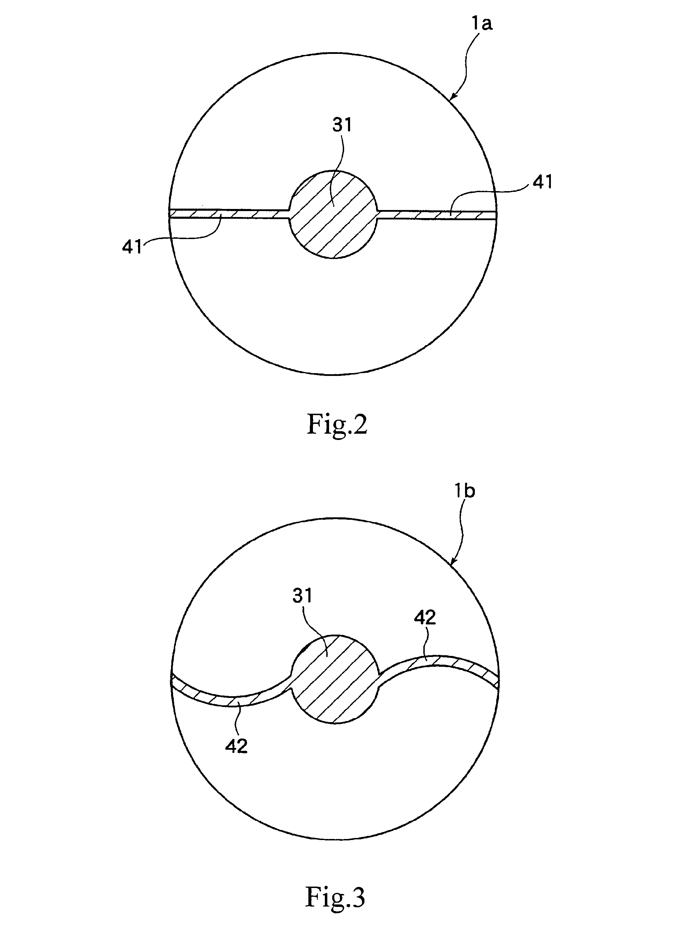

In the bioassay substrates according to the embodiments which will be described below, all the substrates used for information recording, basically, have the above-mentioned configuration in common. Detailed description of the common substrate configuration portion will be omitted below, and Figs. 2 to 13 primarily show the configurations of wiring substrates (or wiring layers).

Now, specific embodiments of the wiring substrate (or wiring layer) constituting the bioassay substrate (hereinafter referred to simply as "the substrate") according to the present invention will be described below, based on Figs. 2 to 17.

The wiring substrate (or wiring layer) is integrated with the above-mentioned information recording substrate 1 (the substrate on which the reaction regions 2 are arranged), to form one bioassay substrate.

First, Fig. 2 is a plan view schematically showing the basic configuration of wiring in a first embodiment (1a) of the wiring substrate (or wiring layer) used in the present invention.

The substrate 1a is provided at its central portion with a circular current passing portion 31 having a predetermined aperture diameter. From the current passing portion 31, two rectilinear feeder wirings 41, 41 are extended toward the outer circumference of the substrate 1a.

While two feeder wirings 41 are formed in Fig. 2, the number of the feeder wirings in the substrate according to the present invention is not limited to this number (two) but can be appropriately selected according to the arrangement configuration of the reaction regions 2 on the substrate or the like factors. Incidentally, the fact that the number of the feeder wirings is not limited to the number as shown applies in the same manner to the following embodiments, and, therefore, the annotating description will be omitted hereafter.

Fig. 3 is a plan view schematically showing the basic configuration of wiring in a second embodiment (1b) of the wiring substrate (or wiring layer) used in the present invention.

In the second embodiment, curved line form feeder wirings 42, 42 are extended from a circular current passing portion 31 located at the center. The curved line form feeder wirings 42 can be appropriately adopted, also in all the embodiments described below.

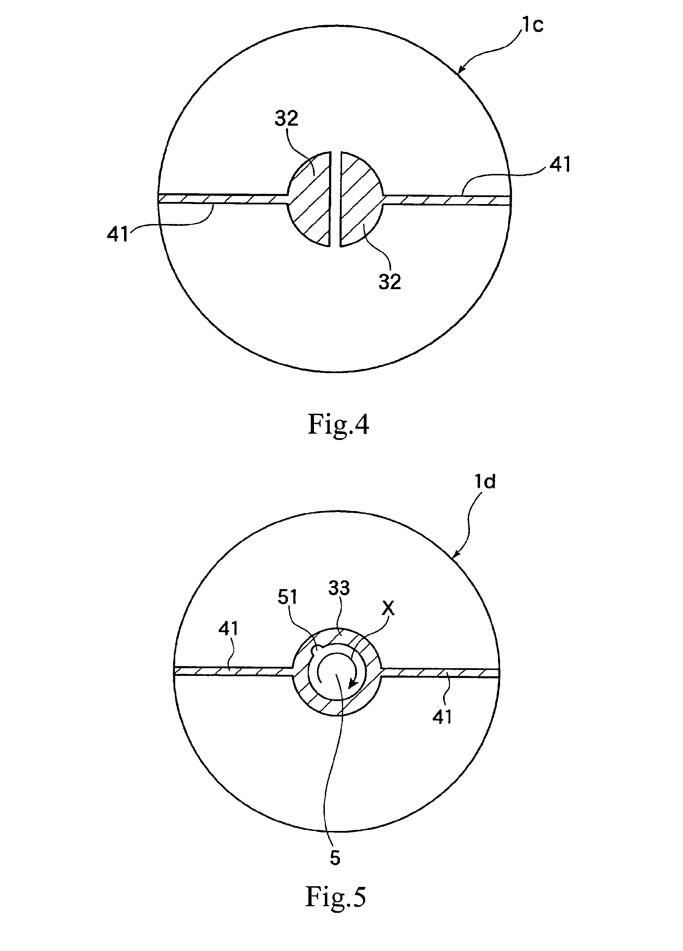

Next, Fig. 4 is a plan view schematically showing the basic configuration of wiring in a third embodiment (1c) of the wiring substrate (or wiring layer) used in the present invention.

The substrate 1c is provided at its central portion with bisected, separate, independent partial current passing regions 32, 32. For example, in the case of impressing a DC electric field, the partial current passing region 32 on one side can be made to function as an anode, while the partial current passing region 32 on the other side can be made to function as a cathode, this point being similarly applicable to the divided form partial current passing regions shown in the drawings described later.

A single feeder wiring 41 is led out from each of the partial current passing regions 32, 32. Incidentally, the number of the divided partial current passing regions 32 is not limited to the shown number of two, and can be appropriately selected according to the purpose. The fact that the number of the partial current passing regions is not limited to the number as shown applies in the same way hereafter, so that the annotating description will be omitted.

Next, Fig. 5 is a plan view schematically showing the basic configuration of wiring in a fourth embodiment (1d) of the wiring substrate (or wiring layer) used in the present invention.

The substrate 1d is provided at its central portion with a current passing portion 33 which is ring-shaped as viewed from above, and two feeder wirings 41, 41 are extended from the current passing portion 33 toward the outer circumference of the substrate.

A circular portion denoted by symbol 5, formed in the inside region of the current passing portion 33, shows a hole (inclusive of a non-through hole, here and hereafter) in which a current passing jig (not shown) or a chucking jig (not shown) of a disk reader/writer or the like is to be inserted. Incidentally, there may also be adopted a configuration in which such a hole 5 is not formed on the inside region of the ring-shaped current passing portion 33.

Here, symbol 51 shown in Fig. 5 is a notch-like recess formed in the hole 5. The recess 51 functions as a positioning portion for preventing the above-mentioned current passing jig or chucking jig from being positionally deviated in the circumferential direction X.

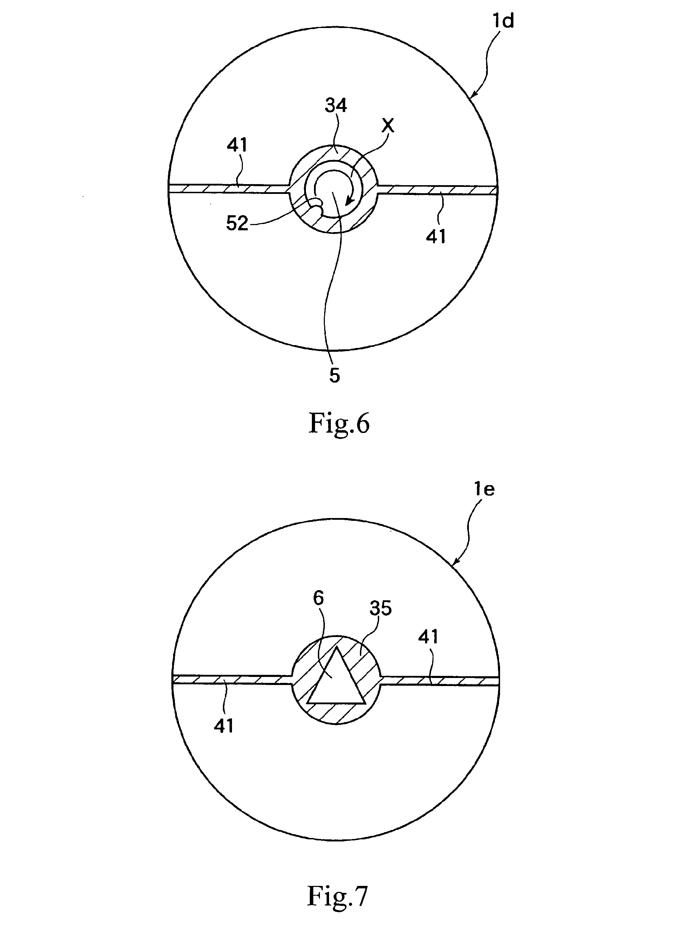

Incidentally, Fig. 6 shows a modified form of the substrate 1d. A projection formed at an inner circumferential portion of the current passing portion 34 shown in Fig. 6 functions as a positioning portion which is the same as above-mentioned.

Next, Fig. 7 is a plan view schematically showing the basic configuration of wiring in a fifth embodiment (1e) of the wiring substrate (or wiring layer) used in the present invention.

A current passing portion 35 provided at a central portion of the substrate 1e is circular in outer shape, and a hole 6 in the shape of an isosceles triangle is formed in a central portion of the substrate 1e. The shape of the hole 6 makes it possible to position the current passing jig or chucking jig, which is to be inserted in the hole 6, in the circumferential direction X (see Figs. 5 and 6).

The shape of the hole 6 having such a positioning function is not limited to the isosceles triangle, and any shape may be appropriately adopted insofar as it determines only one insertion position of the jig. Examples of the applicable shape include a drop shape, a heart shape, and a clover shape.

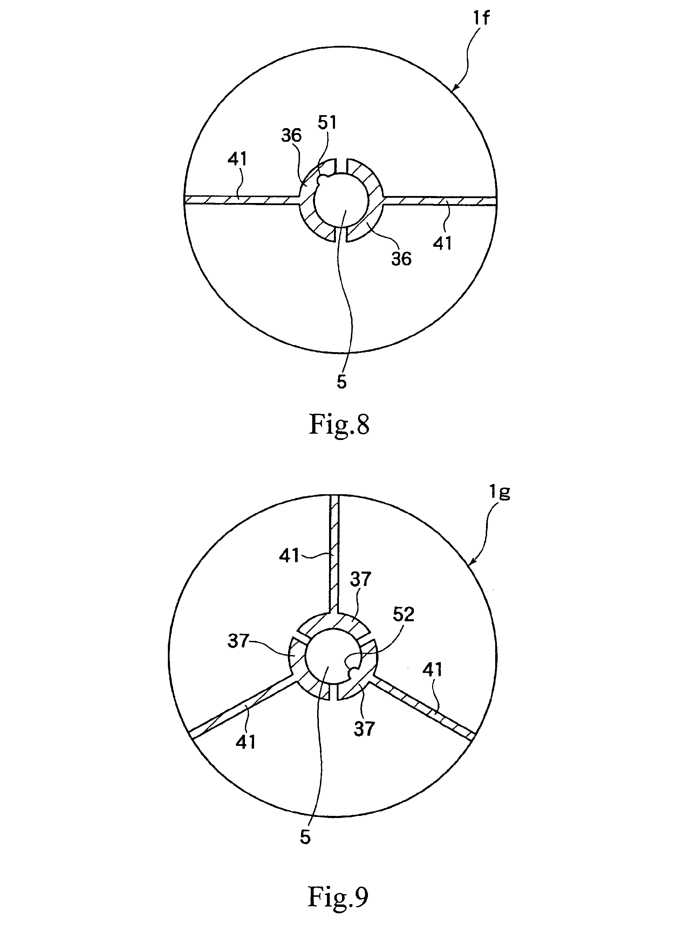

Fig. 8 is a plan view schematically showing the basic configuration of wiring in a sixth embodiment (1f) of the wiring substrate (or wiring layer) used in the present invention.

The substrate 1f is provided at its central portion with a hole 5 having the above-mentioned recess 51 (projection 52 is also applicable), and separate, independent, partial current passing regions 36, 36 divided into roughly semicircular shapes in outlook are formed so as to surround the hole 5. A single feeder wiring 41 is extended from each of the partial current passing regions 36, 36 toward the outer circumference of the substrate If.

Fig. 9 is a plan view schematically showing the basic configuration of a seventh embodiment (1g) of the wiring substrate (or wiring layer) used in the present invention.

The substrate 1g is provided at its central portion with a hole 5 having a projection 52 (recess 51 is also applicable), and separate, independent, partial current passing regions 37, 37, 37 formed as if a ring-shaped current passing portion has been trisected are formed so as to surround the hole 5. A single feeder wiring 41 is extended from each of the partial current passing regions 37, 37, 37 toward the outer circumference of the substrate 1g.

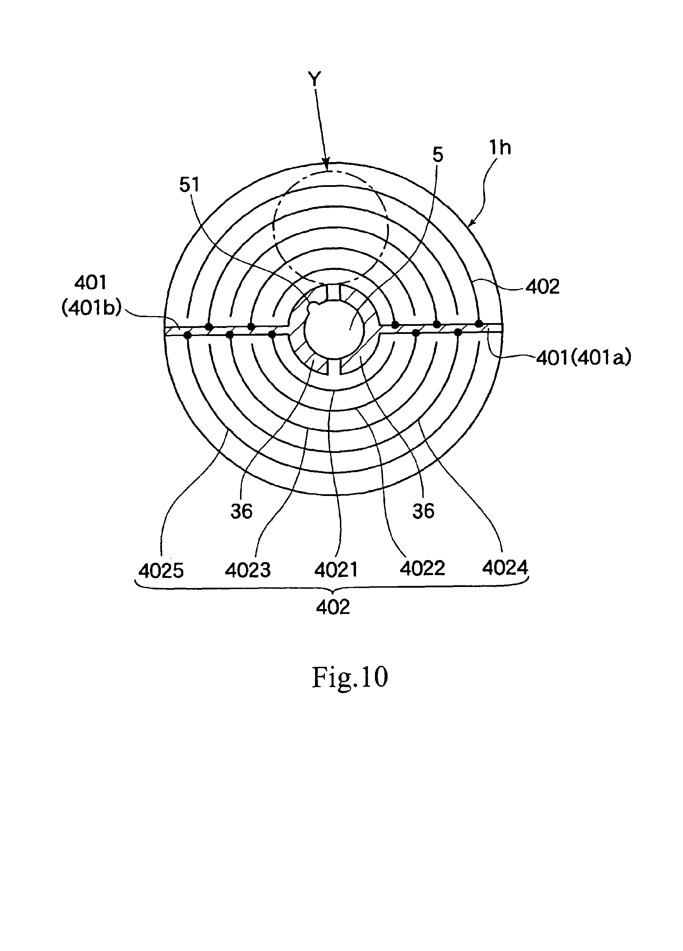

Next, Fig. 10 is a plan view schematically showing the basic configuration of wiring in an eighth embodiment (1h) of the wiring substrate (or wiring layer) used in the present invention.

The substrate 1h includes first wirings 401, 401 extended respectively from bisected partial current passing regions 36, 36 and functioning as main wirings (trunk wirings) for feeding electricity, and a group of second wirings 402 branched from the first wirings 401, 401 and extended so as to draw circular arcs in the circumferential direction.

For example, the width of the first wirings 401 can be designed to be 50 *µ*m, and the width of the second wirings 402 can be designed to be 5 *µ*m (the same applies hereafter). Incidentally, a hole 5 having a recess 51 (projection 52 is also applicable) is formed on the inside of the partial current passing regions 36, 36.

As understood by looking at the first wirings 401 from the center toward the outer circumference of the substrate 1h or from the outer circumference side toward the center side, one-side ends of the second wirings 402 are alternately connected to the first wirings 401 (401a, 401b) (see Fig. 10).

More specifically, one end of the second wiring 4021 is connected to the first wiring 401b, one end of the second wiring 4022 is connected to the first wiring 401a, one end of the second wiring 4023 is connected to the first wiring 401b, one end of the second wiring 4024 is connected to the first wiring 401a, and one end of the second wiring 4025 is connected to the first wiring 401b. The group of the second wirings 402 in such a configuration are in the form of concentric circles, as viewed from the upper side (see Fig. 10).

The second wirings 402 are each connected, for example, to the electrode E1 or E2 in one of the reaction regions 2 arranged, for example, in a substrate region Y indicated by an imaginary-line circle in Fig. 10, and function as feeder wirings for impressing a high-frequency AC electric field, a DC electric field or the like on the electrodes E1 and E2 (the same applies hereafter).

Incidentally, the first wirings 401 extended in the radial direction of the substrate can be used as references for a rotation synchronizing signal, and the second wirings 402 extended in the circumferential direction can be used as references for a tracking signal, which is preferable (the same applied hereafter).

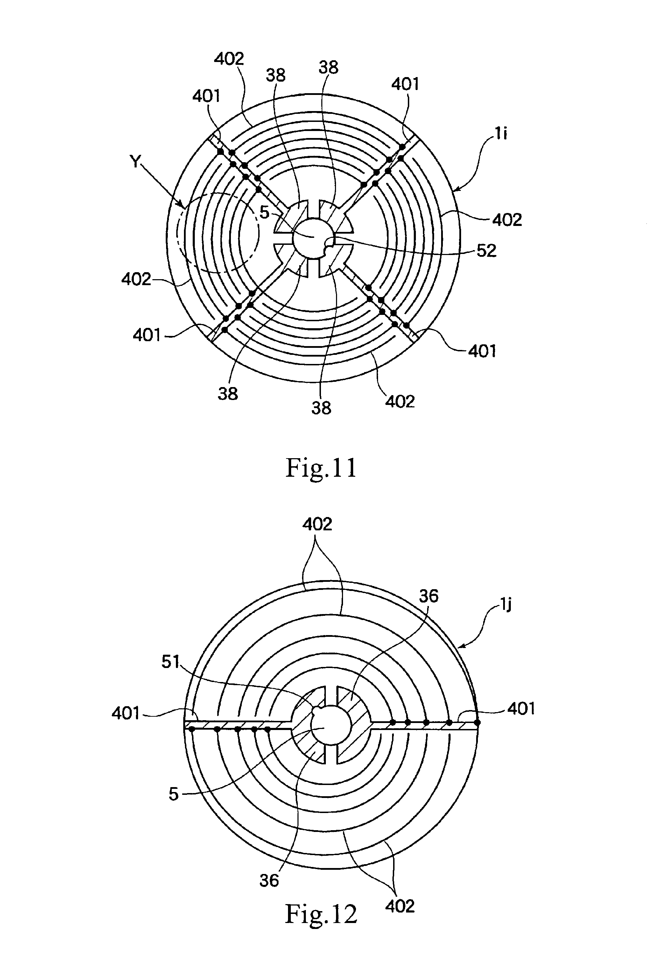

Next, Fig. 11 is a plan view schematically showing the basic configuration of wiring in a ninth embodiment (1i) of the wiring substrate (or wiring layer) used in the present invention.

The substrate 1i has quadrant partial current passing regions 38, 38, 38, 38. For example, in the case of impressing a DC electric field, the partial current passing regions 38, 38, 38, 38 can function as current passing portions of an anode, a cathode, an anode, and a cathode, alternately in the circumferential direction.

First wirings 401, 401, 401, 401 functioning as main wirings (trunk wirings) for feeding electricity are led out and extended respectively from the partial current passing regions 38, 38, 38, 38, and a group of second wirings 402 are branched and extended from each of the first wirings 401 so as to draw circular arcs in the circumferential direction. Incidentally, a hole 5 having a projection 52 (recess 51 is also applicable) is formed on the inside of the partial current passing regions 38, 38, 38, 38.

In this substrate 1i, the configuration of connection of the second wirings 402 to the first wirings 401 or the configuration of branching of the second wirings 402 from the first wirings 401 is the same as in the case of the above-described substrate 1h; in the same manner as in Fig. 10, the group of the second wirings 402 are extended in the pattern of concentric circles, as viewed from above (see Fig. 11).

In addition, the group of the second wirings 402 in the substrate 1i shown in Fig. 11 are led out alternately from the adjacent first wirings 401, 401, and each of the second wirings 402 is connected to only one first wiring 402.

Next, Fig. 12 is a plan view schematically showing the basic configuration of a tenth embodiment (1j) of the wiring substrate (or wiring layer) used in the present invention.

The substrate 1j includes first wirings 401, 401 extended respectively from bisected partial current passing regions 36, 36 and functioning as main wirings (trunk wirings) for feeding electricity, and a group of second wirings 402 branched from the first wirings 401, 401 and extended in the pattern of a spiral line. Incidentally, a hole 5 having a recess 51 (projection 52 is also applicable) is formed on the inside of the partial current passing regions 36, 36.

Here, as easily understood from a comparison between the substrate 1j shown in Fig. 12 and the substrate 1h shown in Fig. 10, the group of the second wirings 402 in the substrate 1j are branched alternately from the first wirings 401 so as to draw semi-circles gradually increased in diameter sequentially from the radially inner side, and they as a whole are in a spiral pattern, in overall outlook. In addition, every one of the second wirings 402 is connected to only one first wiring 401 (see Fig. 12).

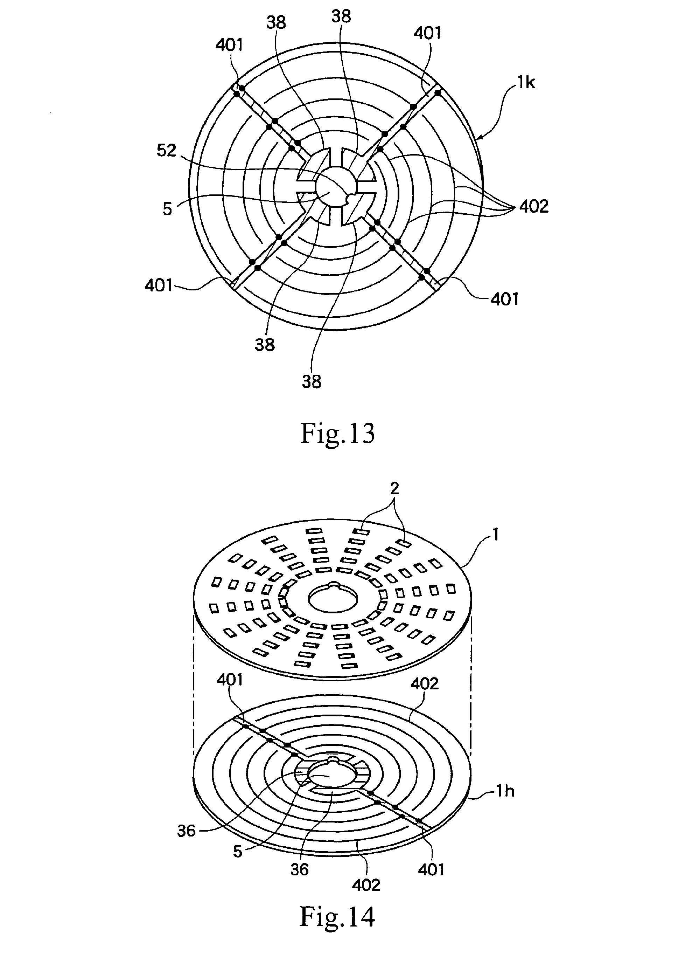

Fig. 13 is a plan view schematically showing the basic configuration of wiring in an eleventh embodiment (1k) of the wiring substrate (or wiring layer) used in the present invention.

The substrate 1k includes four first wirings 401, 401, 401, 401 extended respectively from quadrant partial current passing regions 38, 38, 38, 38 and functioning as main wirings (trunk wirings) for feeding electricity. The substrate 1k also includes a group of second wirings 402 branched from the first wirings 401. The second wirings 402 in the substrate 1k shown in Fig. 13 are formed in a spiral pattern when viewed from above, in the same manner as in the above-described substrate 1j. Incidentally, a hole 5 having a projection 52 (recess 51 is also applicable) is formed on the inside of the partial current passing regions 38, 38, 38, 38.

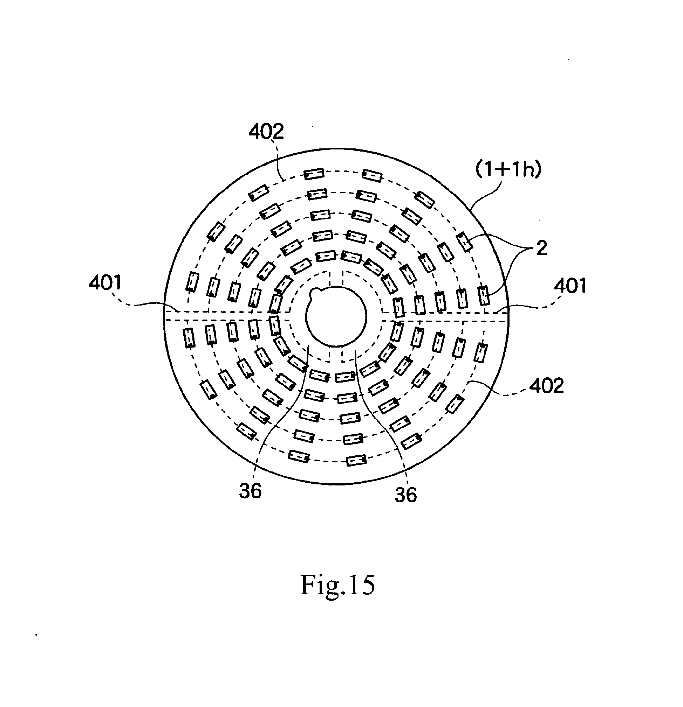

Here, Fig. 14 shows the manner in which an information recording substrate (layer) 1 and a wiring substrate (wiring layer) (the substrate 1h shown in Fig. 10 is adopted here as a typical example), both shown schematically, are laminated integrally, for example, to form one bioassay substrate. Fig. 15 is a plan view, as viewed from the upper side of the substrate (layer) 1, of the condition where the substrate 1 and the wiring substrate (wiring layer) 1h are laminated.

Reaction regions 2 on the substrate 1 are arranged at predetermined intervals so as to be located along second wirings 402 for feeding electricity. In the example shown in Fig. 14, the second wirings 402 of the wiring substrate are extended in a pattern of concentric circles, so that the reaction regions 2 are also arranged in a pattern of concentric circles on the substrate 1. Incidentally, in the case where the second wirings for feeding electricity are extended in a spiral pattern as in the wiring substrates (wiring layers) 1j and 1k shown in Figs. 12 and 13, the reaction regions 2 are also arranged in a spiral pattern on the substrate 1.

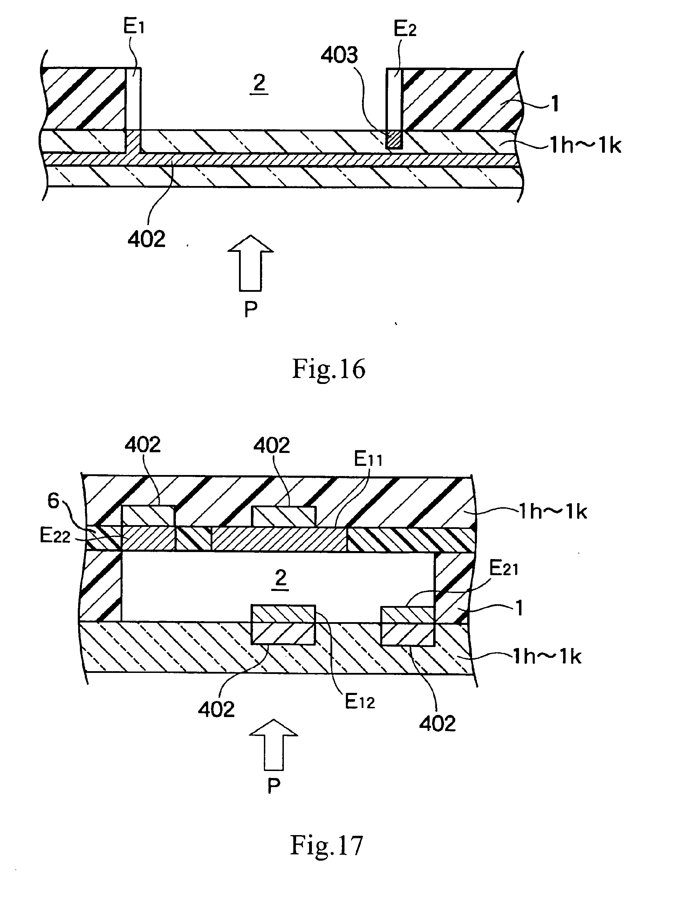

Fig. 16 is a vertical sectional view showing an example of connection of feeder wirings to opposed electrodes E1 and E2 arranged in a reaction region 2.

The electrode E1 on one side is connected to one second wiring 402 of the wiring substrate (wiring layer), as for example denoted by symbols 1h to 1k, disposed on the lower layer side, and, for example in the case of impressing a DC electric field, it is utilized as an anode. The electrode E2 on the other side is connected to an auxiliary wiring 403 extended from the wiring 402 (not shown in Fig. 16) adjacent to and on the outer circumference side or inner circumference side of the above-mentioned second wiring, and, for example in the case of impressing a DC electric field, it is utilized as a cathode.

In addition, a plurality of wiring layers may be provided. For example, as shown in Fig. 17, a configuration can be adopted in which two wiring substrates (wiring layers) are preliminarily provided so as to sandwich a substrate 1 provided with reaction regions 2, one second wiring 402 of the wiring substrate (wiring layer) on the upper layer side is connected to an upper-side electrode E11 fronting on the reaction region 2, and one second wiring 402 of the wiring substrate (wiring layer) on the lower layer side is connected to an electrode E12 disposed on the bottom surface of the reaction region 2 so as to be opposed to the electrode E11.

Then, one second wiring 402 of the wiring substrate (wiring layer) on the lower layer side can be connected to another electrode E21 formed on the bottom surface of the reaction region 2, and one second wiring 402 of the wiring substrate (wiring layer) on the upper layer side can be connected to an electrode E22 (disposed on the upper side of the reaction region 2) for forming an opposed pair of electrodes with the electrode E21.

By adopting such a wiring connection configuration, a high-frequency AC electric field, a DC electric field or the like can be impressed on a medium (not shown) reserved or held in the reaction region 2, between the electrodes E11 and E12 or between the electrodes E21 and E22, for example. Incidentally, symbol 6 in Fig. 17 denotes a substrate laminated on the substrate 1.

The impression of the electric field on the medium reserved or held in the reaction region 2 can be preferably utilized, for example, in the case of stretching or moving a nucleic acid molecule present in the medium by an electrodynamic effect called dielectric migration.

Incidentally, the first wiring 401 being a main wiring connected to each second wiring 402 is exposed from the wiring layer so as to front on the current passing portion (31 to 38) at the center of the substrate, and is connected to the current passing portion at its exposed wiring terminal end portion (not shown).

At least one of a plurality of wiring layers may be formed of an electrode layer (not shown) which is uniform over the whole surface, and at least one of a plurality of wiring layers may be covered with an insulation layer. Incidentally, as the insulation layer, an oxide layer of SiO₂, TiO₂ or the like can be adopted.

Besides, the interactions such as hybridizations having progressed in the reaction regions 2 arranged on the substrate can be detected by use of a known optical or spectroscopic means. In this case, the wiring layer located on the side where an excitation beam (for example, fluorescent excitation beam) P (see Figs. 16 and 17) with a predetermined wavelength for reading (for detection) is incident is composed of an insulation layer which is transparent or semi-transparent to rays in the excitation beam wavelength region.

### Industrial Applicability

The present invention can be utilized as a bioassay substrate, particularly a disk-shaped bioassay substrate, of a DNA chip or the like in which a multiplicity of reaction regions with electrodes are arranged on the substrate.

According to the bioassay substrate of the present invention, in the case of arranging the reaction regions with electrodes on a disk-shaped substrate, feeder wirings connected to the electrodes can be formed on the substrate orderly and in a high density, so that the arrangement density of the reaction regions can be made uniform and higher, irrespectively of the position between the inner and outer circumferences of the substrate, and the wiring resistance can be reduced.

In addition, according to the wiring configuration adopted in the bioassay substrate of the present invention, electricity can be securely fed to the electrodes provided in the reaction regions, irrespectively of the electrode arrangement configuration, so that the degree of freedom in designing can be enhanced.

Besides, the feeder wirings laid on the disk-shaped substrate can be used also as references for a rotation synchronizing signal or references for a tracking signal, which signal is used at the time of reading the recorded information. As a result, the need to provide the substrate with exclusive-use signals or marks, such as a group of pits, a group of bar codes, etc. for obtaining the signals is eliminated, so that the configuration of the substrate can be simplified.

## Claims

1. A bioassay substrate being a disk-shaped substrate having such a configuration that reaction regions to be fields for interactions between substances are arranged and electrodes are provided in said reaction regions, wherein
feeder wirings are extended from a current passing portion provided at a central portion of said substrate, and are connected to said electrodes.

2. The bioassay substrate as set forth in claim 1, wherein said feeder wirings are each comprised of a first wiring extended from said current passing portion toward the outer circumference side, and a second wiring branched and led out from said first wiring.

3. The bioassay substrate as set force in claim 2, wherein said first wiring is extended radially from said current passing portion.

4. The bioassay substrate as set forth in claim 2, wherein said first wiring is extended in the form of a straight line and/or a curved line.

5. The bioassay substrate as set forth in claim 2, wherein a plurality of said first wirings are provided.

6. The bioassay substrate as set forth in claim 2, wherein said second wiring is extended in the circumferential direction.

7. The bioassay substrate as set forth in claim 2, wherein said second wirings are extended in the form of concentric circles or a spiral line.

8. The bioassay substrate as set forth in claim 5, wherein said second wirings are led out alternately from the adjacent first wirings.

9. The bioassay substrate as set forth in claim 5, wherein said second wirings are each connected to only one first wiring.

10. The bioassay substrate as set forth in claim 2, comprising said second wiring which is connected to a plurality of said first wirings.

11. The bioassay substrate as set forth in claim 2, wherein said first wiring is used as a reference for a rotation synchronizing signal.

12. The bioassay substrate as set forth in claim 2, wherein said second wiring is used as a reference for a tracking signal.

13. The bioassay substrate as set forth in claim 1, wherein said current passing portion is comprised of a single current passing region.

14. The bioassay substrate as set forth in claim 1, wherein said current passing portion is circular or ring-like in shape.

15. The bioassay substrate as set forth in claim 1, wherein said current passing portion is divided into a plurality of independent partial current passing regions.

16. The bioassay substrate as set forth in claim 14, wherein at least one first wiring is extended from each of said partial current passing regions.

17. The bioassay substrate as set forth in claim 14, wherein said second wirings are led out alternately from the adjacent first wirings.

18. The bioassay substrate as set forth in claim 1, wherein a hole is formed in a central portion of said current passing portion.

19. The bioassay substrate as set forth in claim 18, wherein said hole is provided with a circumferential-direction positioning portion for a current passing jig and/or a chucking jig to be inserted in said hole.

20. The bioassay substrate as set forth in claim 19, wherein said positioning portion is a recess or a projection formed in said hole.

21. The bioassay substrate as set forth in claim 18, wherein the position or positions in the circumferential direction of said current passing jig and/or said chucking jig to be inserted in said hole are determined by the shape of said hole.

22. The bioassay substrate as set forth in claim 1, wherein said feeder wirings are formed by use of a plurality of wiring layers.

23. The bioassay substrate as set forth in claim 22, wherein said feeder wirings extended in said plurality of wiring layers are exposed to front on said current passing portion, and the exposed wiring terminal portions are connected to said current passing portion.

24. The bioassay substrate as set forth in claim 22, wherein at least one of said plurality of wiring layers is comprised of an electrode layer uniform over the whole area.

25. The bioassay substrate as set forth in claim 22, wherein at least one of said plurality of wiring layers is covered with an insulation layer.

26. The bioassay substrate as set forth in claim 25, wherein said insulation layer is an oxide layer.

27. The bioassay substrate as set forth in claim 22, wherein said wiring layer located on the side where an excitation beam for reading said interactions is incident is comprised of a conductive film which is transparent or semi-transparent to rays in the excitation beam wavelength region.

28. The bioassay substrate as set forth in claim 27, wherein said conductive film is comprised of a film selected from the group consisting of an ITO film, a π electron system conductive polymer film, and a metallic thin film having a thickness of not more than 50 µm.
